# EUROPEAN PATENT APPLICATION

(11) **EP 4 763 351 A1**
(43) Date of publication of application: **24.06.2026**
(21) Application number: 24221090.4
(22) Date of filing: 18.12.2024
(51) Int. Cl.: B05B 12/08, B05B 13/04, C09D 5/00, G01B 11/03, G01B 11/06, G01N 21/84, G01N 33/00

(54) **PROCESS AND DEVICES FOR QUALITY CONTROL DURING VEHCILE MANUFACTURING AND USE**

(71) Applicant: BASF Coatings GmbH, 48165 Münster (DE)
(72) Inventor: ROEHRIG, Ruediger, 48165 Münster (DE); SCHULTE, Christoph, 48165 Münster (DE)
(74) Representative: Coatings IP Association

(57) **Abstract**

The present disclosure relates to a process and a device for quality control during vehicle manufacturing. The disclosure relates to methods, apparatuses, systems, a measuring device, and use of the quality of the coating for monitoring and/or controlling the quality of a coating present on at least a part of the surface of a vehicle during manufacturing of the vehicle.

## Description

### TECHNICAL FIELD

The present disclosure relates to a process and a device for monitoring and/or controlling the property of a coating present on at least a part of a surface of a vehicle during vehicle manufacturing and/or during use of the vehicle.

### TECHNICAL BACKGROUND

Reliable determination of coating property data of coatings (e.g. color data and/or texture data) of vehicles in vehicle production and/or during use of the vehicle may require adherence to given measurement routines defining the measurement locations on the coating as well as the measurement order with respect to such locations.

### SUMMARY

In one aspect disclosed is a method for monitoring and/or controlling the property of a coating present on at least a part of the surface of a vehicle during manufacturing of the vehicle and/or during use of the vehicle, the method comprising the steps of:
- providing a vehicle identifier associated with the vehicle,
- providing - based on the vehicle identifier - coating data associated with the coating, wherein the coating data includes coating property data associated with the property of the coating and measurement position data point(s) associated with the coating property data;
- providing reference coating data set(s) associated with reference coating(s), wherein each reference coating data set includes reference coating property data associated with a property of a reference coating and reference measurement position data point(s) associated with the reference coating property data;
- assigning the coating property data to the reference coating property data based on the measurement position data point(s) associated with the coating property data and the reference measurement position data point(s) associated with the reference coating property data;
- providing the assigned data for determining property data associated with the property of the coating.

In another aspect disclosed is an apparatus for monitoring and/or controlling the property of a coating present on at least a part of the surface of a vehicle during a manufacturing process and/or during use of the vehicle, the apparatus comprising:
- an identifier providing interface configured to provide a vehicle identifier associated with the vehicle,
- a data providing interface configured to provide
   - based on the vehicle identifier - coating data associated with the coating, wherein the coating data includes coating property data associated with the property of the coating and coating measurement position data point(s) associated with the coating property data;
   - reference coating data set(s) associated with reference coating(s), wherein each reference coating data set includes reference coating property data and reference measurement position data point(s) associated with the reference coating property data;
- an assignor configured to assign the coating property data to the reference coating property data based on the measurement position data point(s) associated with the coating property data and the reference measurement position data point(s) associated with the reference coating property data;
- a data providing interface configured to provide assigned data for determining property data associated with the property of the coating.

In another aspect disclosed is a system for monitoring and/or controlling the property of a coating present on at least a part of the surface of a vehicle during a manufacturing process and/or during use of the vehicle, the system comprising:
- at least one measurement device configured to provide coating data associated with the coating, wherein the coating data includes coating property data associated with the property of the coating and measurement position data point(s) associated with the coating property data;
- at least one processor configured to
   - provide reference coating data set(s) associated with reference coating(s), wherein each reference coating data set includes reference property data associated with a property of a reference coating and reference measurement position data point(s) associated with the reference coating property data;
   - assign the coating property data to reference coating property data based on the measurement position data(s) associated with the coating property data and the reference measurement position data point(s) associated with the reference coating property data;
   - provide the assigned data for determining property data associated with the property of the coating.

In another aspect disclosed is a use of property data associated with the property of a coating present on at least one surface of a vehicle as determined according to the methods disclosed herein for monitoring and/or controlling the property of a coating being present on at least a part of the surface of a vehicle, in particular during manufacturing of the vehicle and/or during use of the vehicle.

Any disclosure and embodiments described herein relate to methods, systems, apparatuses, and uses lined out above and vice versa. Advantageously, the benefits provided by any of the embodiments and examples equally apply to all other embodiments and examples and vice versa.

### EMBODIMENTS

The invention relates to the field of quality assurance in automobile production and/or use, in particular to the determination of color consistency and other coating property/ies, such as thickness, of a coating being present on at least a part of a surface of a vehicle.

To determine properties of a coating on a vehicle or vehicle body, a number of measurements have to be performed at given or predefined locations on the coating. The position of the given locations may vary depending on the vehicle type and/or the coating type. In order to obtain reliable coating property data associated with the property, it is important to acquire the coating property data or measurement data used to determine the coating property data at the correct locations on the coating in a given order to allow correct assignment of the coating property data to the measurement location.

The process of manually acquiring measurement data or coating property data at defined measurement positions in a given order is prone to errors, and there is a need for more reliable and accurate determination of the property of the coating based on measurement data acquired at different locations on the coating.

By using measurement position data point(s) to reliably map the coating property data of the coating to reference coating property data of reference coating(s), a more reliable determination of the property/ies of the coating, such as the appearance of the coating, can be achieved. Moreover, this enables to map the coating property data to reference coating property data irrespective of the order used during measurement of the coating property data or measurement data used to determine the coating property data. As a result, the property/ies of the coating can be determined more reliable, hence allowing more reliable monitoring and/or controlling of the property/ies of the coating, for example during manufacturing of the vehicle and/or during use of the vehicle. This way, coatings not meeting target property/ies may be determined more efficiently, allowing to remedy determined property defects more reliably. This allows more efficient manufacturing of the vehicle and/or more efficient maintenance of the coating of the vehicle, reducing the environmental impact associated with the vehicle manufacturing and/or maintenance..

In the following, embodiments of the present disclosure will be outlined by ways of examples. It is to be understood that the present disclosure is not limited to said embodiments and/or examples.

Various units, circuits, entities, nodes, or other computing components may be described as "configured to" perform a task or tasks. Configured to shall recite structure meaning "having circuitry that" performs the task or tasks on operation. The units, circuits, entities, nodes or other computing components can be configured to perform the task even when the unit/circuit/component is not operating. The units, circuits, entities, nodes or other computing components that form the structure corresponding to "configured to" may include hardware circuits and/or memory storing program instructions executable to implement the operation. The units, circuits, entities, nodes or other computing components may be described as performing a task or tasks, for convenience in the description. Such descriptions shall be interpreted as including the phrase "configured to." Any recitation of "configured to" is expressly intended not to invoke 35 U.S.C. § 112(f) interpretation.

In general, the methods, apparatuses, systems, computer elements, nodes or other computing components described herein may include memory, software components and hardware components. The memory can include volatile memory such as static or dynamic random-access memory and/or nonvolatile memory such as optical or magnetic disk storage, flash memory, programmable read-only memories, etc. The hardware components may include any combination of combinatorial logic circuitry, clocked storage devices such as flops, registers, latches, etc., finite state machines, memory such as static random-access memory or embedded dynamic random-access memory, custom designed circuitry, programmable logic arrays, etc.

A vehicle may relate to an automobile such as a car, a van, a minivan, a bus, a SUV (sports utility vehicle); a truck; a semitruck; a tractor; a motorcycle; a trailer; an ATV (all-terrain vehicle); a pickup truck; a heavy duty mover, such as bulldozer, mobile crane and earth mover; an airplane; boats; ships; and other modes of transport that may be coated with at least one coating layer. Preferably, vehicle may relate to an automobile; a truck; a semitruck; a tractor; a motorcycle; a trailer; an ATV (all-terrain vehicle); a pickup truck or a heavy-duty mover. Other terms for vehicle may be automotive, automobile, automotive vehicle, or automobile vehicle.

During a manufacturing process may relate to any point in time during the manufacturing process of a physical vehicle or a vehicle body. Said manufacturing process may be ended when the finished physical vehicle or vehicle body may fulfill the predefined specifications and no further manufacturing or quality control processes may be performed at the manufacturing side. The finished vehicle may be transported to a vendor and/or exported and/or stored. Finished vehicle bodies may be transported to a vehicle assembly plant. During use of the vehicle may relate to any point in time after finishing the manufacturing process. The vehicle may be used by an end-product user.

Coating property data may be associated with one or more property/ies of the coating present on the surface. The coating may comprise one or more coating layers. The coating may comprise colored coating layer(s) and/or transparent coating layer(s). Coating property data may include appearance data such as color data and/or texture data, gloss data, thickness data, roughness data, hardness data and any combinations thereof. Color data may include coordinates in the CIELAB color space (e.g. L*a*b* values) and/or L*C*H* color values,

Coating property data may include measured coating property data. Coating property data may include coating property data determined from measured data, such as reflectance data and/or image data. Coating appearance data may be associated with various points or locations on the surface of the vehicle. The coating appearance data may be acquired at such locations. Measurement data used to determine the coating appearance data may be acquired at such locations. Coating appearance data may be obtained or measured or collected after application of a coating material and/or during and/or after drying of the applied coating material(s) and/or during and/or after curing of the at coating layer(s) obtained from application of the coating material(s). Coating appearance data may include at least one data point. The measured data may be obtained with a measurement device. The measuring device may be a spectrophotometer, such as a multi-angle spectrophotometer. The coating appearance data may be determined using commercially available multi-angle spectrometers such as a Byk- Mac^{®} I or a spectrometer of the XRite MA^{®}-T-family. Reflectance of the respective coating and/or reference coating may be measured for several geometries. In case of effect coatings, texture images (grey scale or color images) may be acquired at several geometries. The multi-angle spectrophotometer may be connected to a computer processor which may be programmed to process the measured reflectance data and texture images, for example by calculating the color data for each measurement geometry from the measured reflectance and/or texture characteristics for a defined measurement geometry. Texture characteristics may refer to the coarseness characteristics and/or sparkle characteristics of an effect coating layer caused by effect pigments, sparkle, or other visual effects of a surface. The determined coating appearance data may be stored on a data storage medium, such as an internal memory or a database prior to providing the determined coating appearance data via the communication interface to the computer processor.

Measurement position data point(s) may indicate one or more positions where the coating property data was acquired or where the measurement data used to determine the coating property data was acquired. Measurement position data point(s) may refer to a specific location or point. The location or point may be relative to the surface of the vehicle or an area thereof. Measurement position data point(s) may be described using coordinate systems based on a defined origin point. Coordinate systems may include cartesian coordinates in a two-dimensional (2D) space (X, Y), in a three-dimensional (3D) system (X, Y, Z), polar coordinates, geographic coordinates or the like. The origin point may include a position on the vehicle body. The origin point may include any position outside of the vehicle body. Measurement position data point(s) may include distance data and/or angle data. The distance data and/or angle data may be associated with one or more further measurement position data point(s). Measurement position data point(s) may be determined based on the position of the measuring device during acquisition of the coating property data or the measurement data. Measurement position data point(s) may be determined based on the position of the measuring device relative to the origin point during acquisition of the coating property data or the measurement data. Measurement position data may be determined based on the position of a user using the measuring device during acquisition of the coating property data or the measurement data. Measurement position data point(s) may be determined based on distances between at least two measurement positions. Distance may be measured using sensors such as accelerometers. Measurement position data may be determined using a fixed start point based on speed, direction, and elapsed time. Measurement position data point(s) may be determined by an indoor positioning system (IPS). IPS may be a network of devices used to locate people or objects. The measurement position data may be acquired using wireless technologies such as Wi-Fi base positioning systems, Bluetooth, or the like. Measurement position data point(s) may be acquired using magnetic positioning.

Reference coating data set(s) may relate to target property data associated with target property/ies. The reference coating data set may be associated with a given vehicle, a given vehicle type and/or a given coating type The reference coating data sets may include reference coating property data and reference measurement position data point(s) the reference coating property data was acquired or measurement data used to determine the reference coating property data was acquired. The reference coating property data may be location dependent, e.g. may vary with respect to different measurement locations on the coating. Hence, the reference coating property data may include different property data for at least a part of the reference measurement position data points. The reference coating property data may be location independent, e.g. may be constant with respect to different measurement locations on the coating. Hence, the reference coating property data may include identical property data per reference measurement position data point. Reference coating data sets may include data related to properties of the vehicle such as vehicle specifications (height, width, length, weight, or the like). Reference coating data sets may include data related to identification information such as a vehicle ID or a vehicle type. Reference coating data sets data may include data related to the visualization of the vehicle such as 3D representations or butterfly view of the vehicle. Reference coating data sets may include data related to production data of the vehicle. Reference coating data sets may include data related to the producer, such as production location, producer name, producer brand or producer identifier. Reference coating data sets may include reference coating property data associated with reference measurement position data point(s). The reference coating property data may be associated with at least one property of a reference. The reference coating may include a coating having target property/ies. Reference coating data sets may include data related to the reference coating such as color number(s), color code(s), bar code(s) and/or unique database ID associated with the reference coating and/or coating material(s) used to prepare the reference coating, the layer structure of the reference coating, the wet or dry film thickness(es) of the reference coating, or any combinations thereof.

Reference coating property data may relate to target coating property data associated with a reference coating or target coating. Reference coating property data may relate to predefined coating property data such as predefined color data, gloss data, appearance data, thickness data, roughness data, hardness data, texture data. Reference coating property data may relate to predefined deviation(s) from the target property/ies. Deviations may include single data point(s) and/or threshold(s) and/or range(s). Predefined deviation(s) may be selected from a predefined color difference, a predefined appearance tolerance, a predefined gloss tolerance, a predefined thickness tolerance, a predefined roughness tolerance or a predefined hardness tolerance or any combinations thereof and any single tolerances thereof. The reference coating data set(s) may be associated with the vehicle identifier or a vehicle type identifier the vehicle identifier is associated with. The reference coating data set(s) may be associated with coating identifier(s) associated with the reference coating. Reference coating property data may be stored in a database which may be accessed via a communication interface. Reference coating property data may be associated with reference measurement position data point(s). The data point(s) may indicate the position(s) on the coating the reference coating property data was acquired or measurement data used to determine the reference coating property data was acquired.

Reference measurement position data point(s) may indicate the position on the reference coating where the reference coating property data was acquired or where measurement data used to determine the reference coating property data was acquired. The reference measurement position data point(s) may relate to given or predefined measurement positions. Reference measurement position data point(s) may refer to a set of target positions on the coating to be used to reliably determine the property of the coating of a vehicle.

In one embodiment the coating property data includes appearance data such as color data and/or texture data, gloss data, appearance data, thickness data, roughness data, hardness data and any combinations thereof.

In one embodiment the measurement position data point(s) indicate(s) one or more position(s) on the coating used to acquire the coating property data. In another embodiment the measurement position data point(s) indicate(s) one or more position(s) on the coating used to acquire measurement data for determining the coating property data. The coating property data may be determined from measurement data acquired at such position(s) on the coating. The coating property data and/or the measurement data may be acquired using measurement devices, such as spectrophotometers, cameras, glossmeters or the like. The coating property data and/or the measurement data may be acquired at such position(s) under different illumination conditions and/or measurement geometries.

In one embodiment, the measurement position data points may be associated with data indicating an order of such measurement position data points relative to each other. The order may indicate a start point and an end point.

In one embodiment the reference measurement position data point(s) indicate(s) one or more position(s) on the coating used to acquire the reference coating property data or used to acquire measurement data for determining the reference coating property data. The reference measurement position data point(s) may relate to given or predefined or target positions. Reference measurement position data point(s) may relate to given reference position(s). Reference measurement position data point(s) may indicate target location(s) for acquiring the coating property data and/or measurement data used to determine the coating property data. The coating property data and/or the measurement data may be acquired based on the reference measurement position data point(s).

In one embodiment, the reference measurement position data points may be associated with data indicating an order of such reference measurement position data points relative to each other. In another embodiment the reference coating data set(s) is/are provided based on the vehicle identifier and/or based on the coating measurement position data point(s). The vehicle identifier may be provided by a user via an interface. The vehicle identifier may be used to gather the reference coating data sets. The vehicle identifier may be associated with a coating identifier associated with the coating. The reference coating data sets may be gathered based on the coating identifier associated with the vehicle identifier. The reference coating data sets may be stored on a data storage medium, such as a database and may be gathered based on the vehicle identifier and/or the coating identifier the vehicle identifier is associated with.

Providing the reference coating data set(s) based on the coating measurement position data point(s) may include calculating deviations between at least a part of measurement position data point(s) and at least a part of reference measurement position data point(s) included in the reference coating data sets. The reference measurement position data point(s) may be included in reference coating data sets. The reference coating data sets may be stored on a data storage medium, such as a database. Deviations may be determined based on distance vectors (e.g. sample distance vectors) between measurement position data point(s) and distance vectors (e.g. reference distance vectors) between reference measurement position data point(s). The distance vectors may indicate a distance between respective position data point(s). The reference coating data sets may include such reference distance vectors. The reference distance vector(s) may be determined based on the reference measurement position data point(s). The reference coating data set(s) may be determined by determining a minimum deviation between sample distance vectors and reference distance vectors and selecting the reference coating data set(s) associated with the minimum distance. The deviation may include an absolute deviation between the sample distance vectors and the reference distance vectors. The reference coating data set(s) associated with the minimum absolute distance may include reference coating data set(s) including reference distance vectors associated with the minimum deviation The minimum deviation may be determined by summing up the determined deviations between at least a part of the distance vectors and selecting the minimum absolute sum. The minimum deviation may be determined by comparing each deviation and selecting the combination of reference distance vectors having a minimum deviation from the sample distance vectors. The deviation may be determined for at least a part of the reference coating data sets, such as for at least a part of the reference coating data sets stored in the database. The reference coating data sets may be gathered from a database based on the reference measurement position data point(s) associated with the determined minimum.

In another embodiment the reference coating data set(s) include(s) threshold value(s) for assigning coating property data to the reference property data and/or threshold value(s) associated with a difference between the coating property data and the reference coating property data. The threshold value(s) for assigning coating property data to the reference coating property data may include threshold value(s) related to deviation(s) between measurement location data point(s) and reference measurement location data point(s). Reference coating property data may include predefined threshold value(s). Threshold value(s) may be color thresholds and/or appearance thresholds and/or deviation thresholds. The threshold value(s) may be applied to determine whether to assign the coating property data to the reference property data or vice versa. The threshold value(s) may be used to classify the property of the coating and/or the matching of the coating measurement position data point(s) to the reference measurement position data point(s). Since a high deviation may result in the provision and/or use of non-matching reference coating property data (e.g. reference coating property data associated with a different vehicle type and/or a different coating type), such predefined tolerance range may ensure that suitable (e.g. best matching) reference coating property data is assigned to the coating property data to allow reliable determination of the property/ies of the coating.

In another embodiment assigning the coating property data to the reference coating property data includes
- matching the measurement position data point(s) to reference measurement position data and
- assigning the coating property data associated with the measurement position data point(s) matching the reference data point(s) to reference property data associated with such reference data point(s).

Matching the measurement position data point(s) to reference measurement position data point(s) may be performed before assigning the coating property data to reference property data. Matching the measurement position data point(s) to reference measurement position data point(s) may include minimizing deviation(s) between the measurement position data point(s) and the reference measurement position data point(s). Matching may include determining the similarity between the measurement position data point(s) and the reference measurement position data point(s) or *vice versa.* Matching may include permuting the order of the measurement position data point(s) and determining the distance to a given reference measurement position data point within the reference measurement position data set. The measurement position data point(s) associated with the lowest distance may be selected as measurement position data point matching the given reference measurement position data point Matching may include determining distance vectors between measurement position data points. The distance vectors may be compared to distance vectors associated with reference measurement position data points, as previously described. The minimum deviation between the distance vectors may be determined, for example as previously described. The measurement position data points associated with the distance vectors having the lowest distance to distance vectors associated with the reference measurement position data point(s) may be matched to the reference measurement position data point(s).ln another embodiment the coating property data is assigned to the reference coating property data if at least part of the measurement position data point(s) match(es) to at least part of the reference measurement position data point(s). By mapping the measurement position data point(s) to the reference measurement position data point(s) the similarity between the position data point(s) may be determined. Since the property data is highly dependent on the measurement position used to acquire and/or determine such data, the mapping ensures a degree of similarity between the position data associated with the coating property data and the position data associated with the reference coating property data, hence avoiding that property data associated with different measurement positions is used to determine the property/ies which leads to incorrect property determination. This way, the accuracy of the determined property/ies can be improved independent of the order of the coating property data points included in the coating property data. This way, the efficiency of the manufacturing of the vehicle and/or the maintenance of coating during the use of the vehicle may be improved without requiring a defined order of coating property data points to be included in the coating property data. This allows to reliably and accurately determine the property/ies of coatings irrespective of the acquisition process used to acquire the coating property data and/or measurement data used to determine the coating property data. This way, the flexibility in the data acquisition process can be improved without negatively influencing the reliability and accuracy of the property determination process. In another embodiment the coating property data is assigned to the reference coating property data if the deviation(s) between at least a part of the measurement position data point(s) and at least a part of the reference measurement position data point(s) is/are below a given threshold value. In another embodiment the coating property data is assigned to the reference coating property data if the deviation(s) between at least a part of the measurement position data point(s) and at least a part of the reference measurement position data point(s) is/are within a given range. The deviations may be determined per position data point. The threshold value or range may be included in the provided reference coating data sets. The threshold value or range may be included in the reference coating property data and/or the reference measurement position data point(s). By using tolerance ranges or thresholds slight variations in the measurement locations between sample measurements (.e.g. measurements performed on the coating to be evaluated) and reference measurements may be considered while ensuring that such variations do not result in a negative influence on the determined property/ies due to the location dependency of the coating property/ies. The tolerance ranges or thresholds may signify an area located around the reference measurement positions associated with the reference measurement position data points. The tolerance range or threshold may signify the degree of deviation that is allowable from the reference measurement position data points without significantly influencing the property/ies of the coating. This ensures that the coating property/ies can be reliably and accurately determined based on the assignment of the coating property data of the coating to the reference coating property data of the reference coating.

In another embodiment at least a part of the coating property data is not assigned to the reference coating property data if the deviation(s) between at least a part of the coating measurement position data point(s) and the reference measurement position data point(s) is/are above given threshold value(s). The given threshold value(s) may be included in the reference coating data sets. The given threshold value(s) may be included in the reference coating property data or the reference measurement position data point(s). The deviation(s) may be determined per position data point. If the deviation is above a given threshold, the coating property data point associated with such deviation may not be suitable for reliable determination of the quality of the coating due to the location dependency of the property data.

If at least a part of the coating property data is not assigned to the reference coating property data, data indicating non-assignment of the coating property data to reference property data may be generated and provided. The data indicating non-assignment may be provided via a communication interface. The data indicating non-assignment may include message data. The message data may be provided via the communication interface for display. The message data may include the non-assigned coating property data point(s) and/or the non-matching measurement position data point(s). The message data may prompt the user to provide updated coating property data. The updated coating property data may be provided by acquiring new measurement data, determining updated coating property data based on the measurement data and providing the updated coating property data. The updated coating property data may be provided by acquiring updated coating property data and providing the updated coating property data. The data indicating non-assignment may include control data configured to control electronic components providing visual feedback to a user. The electronic components may be included in the measurement device used to acquire the coating property data or the measurement data. This way, the user may receive feedback and may initiate acquisition of updated coating property data or measurement data.

In another embodiment the method further includes a step of determining - based on the assigned data - the property data and providing the determined property data. The property data may be determined by comparing the reference coating property data to assigned coating property data or vice versa, in particular on data point level. The property data may be determined by comparing color data and/or texture data included in the reference coating property data with respective assigned color data and/or texture data included in the coating property data. The property data may be determined by comparing thickness data included in the reference coating property data with assigned thickness data included in the coating property data. Determining the property data by comparing the color data may include determining a color distance (also denoted as color tolerance) between the reference color data and the assigned color data included in the coating property data and comparing the determined color distance to predefined threshold value(s). The color distance may be determined using a color tolerance equation. The color distance may be determined per measurement angle associated with the color data. The color distance may be determined using a color tolerance equation, such as the Delta E (CIE 1994) color tolerance equation, the Delta E (CIE 2000) color tolerance equation, the Delta E (DIN 99) color tolerance equation, the Delta E (CIE 1976) color tolerance equation, the Delta E (CMC) color tolerance equation, the Delta E (Audi95) color tolerance equation or the Delta E (Audi2000) color tolerance equation. The property of coating may correspond to a binary classifier, discriminating between "OK" and "not OK". "OK" may be associated with a determined color distance being below a given threshold value while "not OK" may be associated with a determined color distance being above a given threshold value. Likewise "OK" may be associated with a determined color distance being above a given threshold value while "not OK" may be associated with a determined color distance being below a given threshold value. The binary classifier may be indicative of or may signify the property of the coating being present on the surface of the vehicle. The binary classifier may be indicative of or may signify the quality of the coating in terms of optical appearance, thickness, gloss, roughness and/or hardness.

The property data may include the property/ies or property type(s) associated with deviation(s) with respect to the assigned reference property/es or property type(s) that are below the threshold value(s) and the determined deviation between the coating property data and the assigned reference coating property data. The property data may further include position data point(s) and/or deviation(s) between the position data point(s).

Providing the determined property data may include providing the determined property data via a communication interface. Providing the determined property data may include providing the determined property data via a communication interface for display. Providing the determined property data may include generating monitoring data for monitoring the property of the coating. The monitoring data may be generated based on the deviation of the coating property data from respectively assigned reference coating property data. This deviation may indicate or signify the condition or state of the coating. The monitoring data may include data indicating new provisioning of coating property data to reevaluate the property of the coating. The data indicating new provisioning may include the vehicle identifier and a time point for providing new coating property data. The data may further include the determined property. By generating monitoring data based on the determined property of the coating, the property of the coating may be more reliably monitored. This way, coating maintenance may be performed in a more efficient manner, avoiding repair of large areas of the coating and allowing to reduce the environmental impact associated with the maintenance by reducing the amount of coating materials and energy consumption as well as the waste generation associated with the maintenance.

Providing the determined property data may include generating control data for controlling the property of the coating. The control data may be generated based on the deviation of the coating property data from respectively assigned reference coating property data. The control data may include process data associated with a process for modifying the coating. Modifying the coating may include removing the coating and recoating the surface to prepare a new coating, Modifying the coating may include overcoating the coating to prepare a new coating on the existing coating. The process data may include a process identifier associated with the process. By generating control data based on the determined property of the coating, the property of the coating may be adjusted to match a given target property, e.g. the property of a reference coating associated with the reference coating property data. This way, it may be ensured that the coating fulfils required property/ies.

### BRIEF DESCRIPTION OF THE SEVERAL VIEWS OF THE DRAWINGS

To easily identify the discussion of any particular element or act, the most significant digit or digits in a reference number refer to the figure number in which that element is first introduced.
- FIG. 1: illustrates an example for acquiring measurement data being indicative of at least one coating property of a coating of a vehicle and measurement position data points associated with measurement positions used to acquire the measurement data by a measuring device.
- FIG. 2: illustrates a flow chart of an example method for monitoring and/or controlling the property of a coating present on at least part of the surface of a vehicle during manufacturing of the vehicle and/or during use of the vehicle.
- FIG. 3: illustrates a first example for providing reference coating data sets including reference coating property data and reference measurement position data points associated with the reference coating property data.
- FIG. 4: illustrates a second example for providing reference coating data sets including reference coating property data and reference measurement position data points associated with the reference coating property data.
- FIG. 5: illustrates an example of assigning coating property data to reference coating property data based on measurement position data points associated with the coating property data and reference measurement position data points associated with the reference coating property data.
- FIG. 6: illustrates an example of determining the property a coating of a vehicle based on coating property data assigned to reference coating property data.

### DETAILED DESCRIPTION

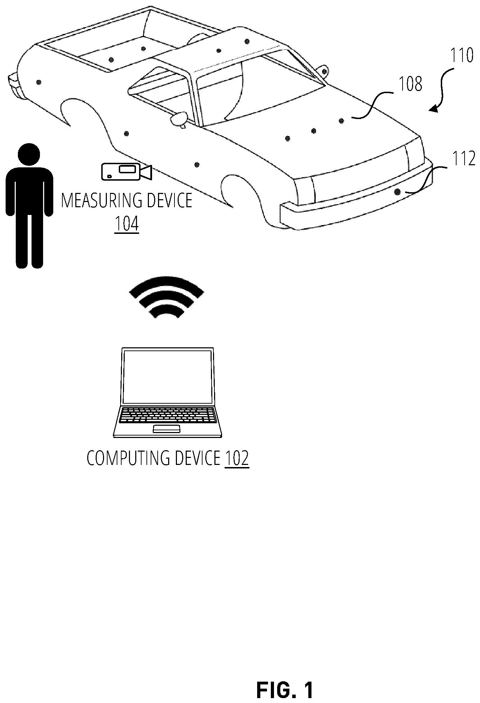

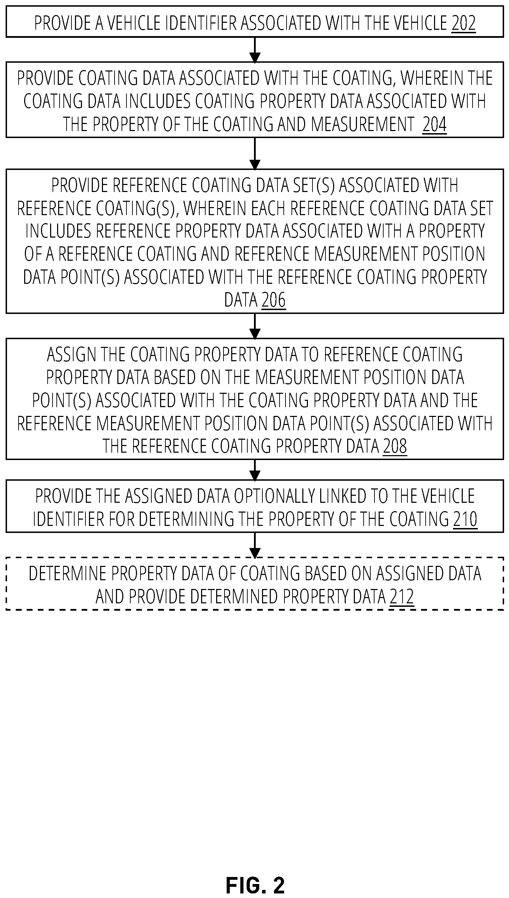

FIG. 1 illustrates an example for obtaining measurement data and associated measurement position data points by a measuring device. The measurement data may be being indicative of at least one coating property of a coating present on at least a part of the surface of a vehicle. The measurement data may include coating property data. The coating property data may be determined based on the measurement data. The measurement position data points may indicate the measurement positions the measurement data was acquired. The vehicle 110 may be a vehicle or a part thereof, such as a vehicle body or a vehicle component. The vehicle or the part thereof may comprise at least one coating present on at least a part of the surface of the vehicle or the part thereof. The coating may be prepared by applying one or more coating material(s) to the surface of the vehicle or the part thereof and drying and/or curing the applied coating material(s). The applied coating material(s) may be cured jointly or separately. At least a part of the applied coating materials may be cured jointly while at least another part of the applied coating materials may be cured separately. The measuring device 104 may be in communicatively coupled with a computing device 102. The computing device may be configured to determine the property of the coating based on the acquired measurement data. The computing device may be configured to perform the methods illustrated in FIG. 2 to FIG. 6.

Reference measurement position data may be provided. The reference measurement position data may indicate where the measurement data is to be acquired. Reference measurement position data may include given locations on the coating where the measurement data is to be acquired. Reference measurement position data may include predefined angles to acquire the measurement data. Reference measurement position data may include a given start point. The start point may indicate the first or start position where the acquisition of the measurement data is to be started. The reference measurement position data may be shown to the user by displaying a 3D rendered representation of the vehicle including the reference measurement positions on the screen of computing device. This may allow to illustrate the respective reference measurement positions for acquiring the measurement data to the user, enabling more efficient and reliable data acquisition by the user. The reference measurement position data may be shown to the user by displaying the points to be measured in a flat pattern view or a butterfly view. The reference measurement position data may be shown to the user by displaying a table including said reference measurement positions. The reference measurement position data may be provided to the computing device. The computing device may display the 3D representation of the vehicle, the 2D representation of the vehicle or the table.

A user may manually acquire the measurement data. The measurement data may be acquired based on the reference measurement positions. By acquiring the measurement data at the reference measurement positions, it can be ensured that the property/ies of the coating present on the surface of vehicle can be reliably determined in comparison to a given reference coating since the property/ies may vary depending on the measurement position used to acquire the measurement data. The user may be an employee of a car manufacturer. The user may acquire the measurement data at predefined positions indicated as dots on the vehicle. The measurement data may be acquired automatically. An automated measuring device may be controlled to acquire the measurement data at the predefined positions. The predefined positions may be provided to the user via a 2D representation of the vehicle surface. The predefined positions may be provided as coordinates to the measuring device. The acquired measurement data may be used to determine the coating property data. The coating property data may include color data, texture data, thickness data, gloss data, roughness data, hardness data, any combinations thereof or any single data thereof. The color data may include color space data, such as CIEL*a*b* values and/or L*C*h* values. The coating property data may be associated with measurement position data points. The coating property data may include one or more data point(s), each data point being associated with a measurement position data point associated with the measurement position used to acquire the measurement data.

Measurement data may be acquired in random order by the measuring device at locations on the coating defined by the reference measurement positions. If a starting point is defined by the reference measurement positions, the measurement data may be acquired at the starting point followed by acquisition of sample measurement data at further locations defined by said reference measurement positions in random order.

The measuring device may be a color measuring device. The measuring device may be a spectrophotometer, such as a multi-angle spectrophotometer and/or a colorimeter. The measuring device may be a camera, a gloss meter and/or a thickness gauge. The measuring device may be configured to acquire measurement data indicative of the color of the coating, the texture of the coating, the gloss of the coating, the thickness of the coating, the roughness of the coating and/or the hardness of the coating. Measurement data may include measurement position data point(s) shown as dots in Fig. 1. Measurement position data points may indicate the position of the acquisition of the measurement data on coating. The measuring device may be equipped with an accelerometer. The measuring device may include any other sensor capable of measuring a distance. The measuring device may measure the distance between positions. Measurement position data may be measured using indoor navigation systems. The measuring device may be configured to determine the coating property data based on the acquired data. The measuring device may be configured to provide data, such as measurement data and associated measurement position data points and/or coating property data and associated measurement position data points, for example via a communication interface.

The measuring device may be connected to the computing device via a wired and/or wireless connection such as one of ethernet, USB, LAN, WLAN and the like. Wireless communication using, for example, WLAN, Wi-Fi, cellular, and/or Bluetooth. Measurement data and/or determined coating property data may be provided to the computing device. The computing device may be configured to store received data, such as measurement data and/or coating property data, on at least one memory. The computing device may be configured to determine coating property data based on the received measurement data. For instance, color data may be determined from the received measurement data, such as reflectance spectra.

FIG. 2 illustrates a flow chart of an example method for monitoring and/or controlling the property of a coating present on at least part of the surface of a vehicle during manufacturing of the vehicle and/or during use of the vehicle. The vehicle may be a vehicle or a part thereof, such as a component thereof. The component may be a door, a bumper, a fender, a trunk or the like.

The surface of the vehicle may be coated with a coating (e.g. sample coating) comprising at least one coating layer. The coating may be produced by applying at least one coating material to the respective surface and drying and/or curing the applied coating material to form a respective coating. The property may include appearance, gloss, thickness, roughness and/or hardness. Appearance may denote the visual impression of the coating perceived by a human observer. Appearance may include the color and/or the texture of the coating.

Coating data associated with the coating may be provided. The coating data may be provided based on a vehicle identifier associated with the vehicle. The vehicle identifier may be a unique identifier uniquely identifying the vehicle within the vehicle production. The vehicle identifier may include letters and/or numbers and/or symbols. The vehicle identifier may be provided by a user via a user interface. The vehicle identifier may be provided from a sensor reading an identification element physically attached to the vehicle. The identification element may encode the vehicle identifier. The identification element may comprise a code, such as a bar code, a QR code or the like, encoding the vehicle identifier.

The coating data may include coating property data and measurement position data point(s) associated with the coating property data. The coating property data may include color data, texture data, gloss data, thickness data, hardness data and/or roughness data. The coating property data may include color data points, texture data points, gloss data points, thickness data points, hardness data points and/or roughness data points. The measurement position data point(s) may indicate one or more position(s), where the coating property data or measurement data used to determine the coating property data was acquired. The measurement position data points may include Cartesian coordinates, geographic coordinates or indoor local Cartesian coordinates. The measurement position data points may be associated with order data indicating the order of measurement position data points, e.g. the order of acquisition of the measurement position data points during acquisition of the measurement data. A given measurement position data point may be associated with the coating property data at such measurement position data point and order data indicating the order of such measurement position data point with respect to other measurement position data points associated with the coating property data. A measurement position data point may be associated with a coating property data point indicating a coating property at the position on the coating indicated by the measurement position data point.

Reference coating data set(s) associated with reference coating(s) may be provided. The reference coating data set(s) may include reference coating property data and reference measurement position data point(s) associated with the reference measurement position data point(s). The reference coating data set(s) may include the reference coating property data and associated reference measurement position data points in a tabular data structure. The reference coating property data may include color data, texture data, gloss data, thickness data, hardness data and/or roughness data. The reference coating property data may include color data points, texture data points, gloss data points, thickness data points, hardness data points and/or roughness data points. The reference measurement position data points may include Cartesian coordinates, geographic coordinates or indoor local Cartesian coordinates. The reference measurement position data points may include mandatory position data points and optional position data points. Mandatory position data points may include position data points that need to be matched to a sufficient degree to measurement position data points, e.g. the deviation between the coating measurement position data points and such mandatory reference measurement position data point(s) needs to be below a given threshold value. By defining mandatory reference measurement position data points, a sufficient degree of similarity between data points associated with property data being indicative of the property of the coating may be achieved, hence ensuring a reliable determination of the property of the coating based on the provided coating data and the reference coating data set(s). Reliable determination of the property of the coating allows more efficient repair and/or maintenance processes with respect to the coating, allowing to reduce the overall environmental impact associated with repair and/or maintenance processes by reducing the number of repair processes or by increasing the maintenance intervals due to more reliable determination of the property of the coating.

The reference measurement position data points may be associated with order data indicating the order of reference measurement position data points. A given reference measurement position data point may be associated with the reference coating property data at such reference measurement position data point and order data indicating the order of such reference measurement position data point with respect to other reference measurement position data points associated with the reference coating property data. A reference measurement position data point may be associated with a reference coating property data point indicating a reference coating property at the position on the reference coating indicated by the reference measurement position data point.

The reference vehicle data may further include a virtual representation of the vehicle such as a 3D representation and/or flat pattern data associated with a 2D representation of the vehicle.

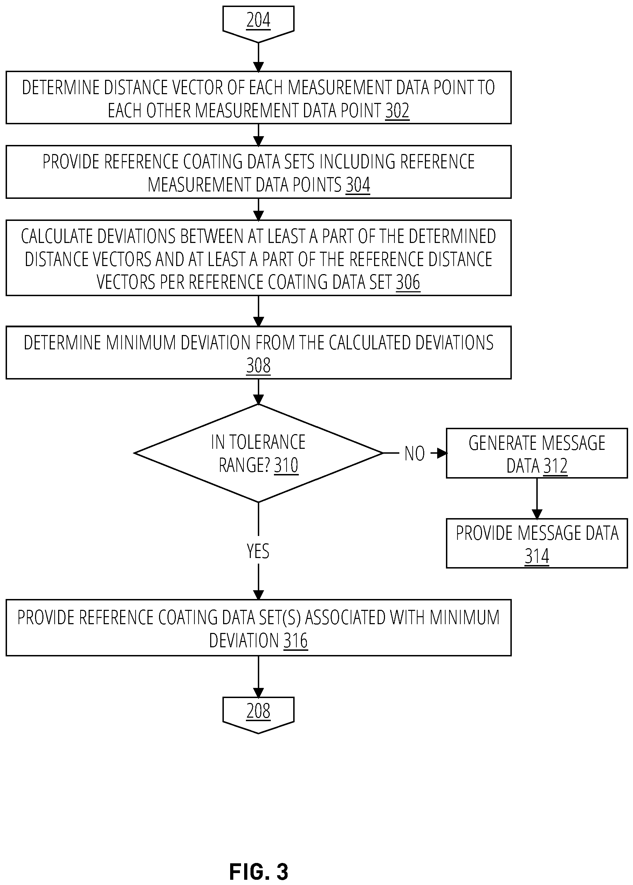

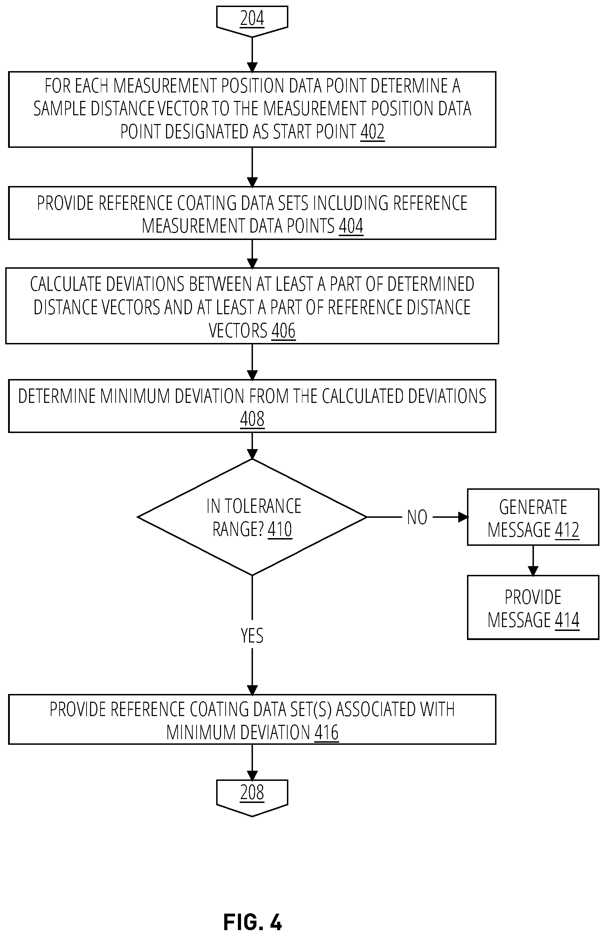

The reference coating data set(s) may be provided based on the vehicle identifier, such as a VIN number. The vehicle identifier may be used, for example by the computing device 102, to gather the reference coating data set(s) from a database storing reference coating data sets interrelated with vehicle identifiers. The vehicle identifier may be provided by a user. The vehicle identifier may be provided via a sensor reading an identification element. The reference coating data set(s) may be provided based on the coating measurement position data points as described in the context of FIG. 3 and FIG. 4.

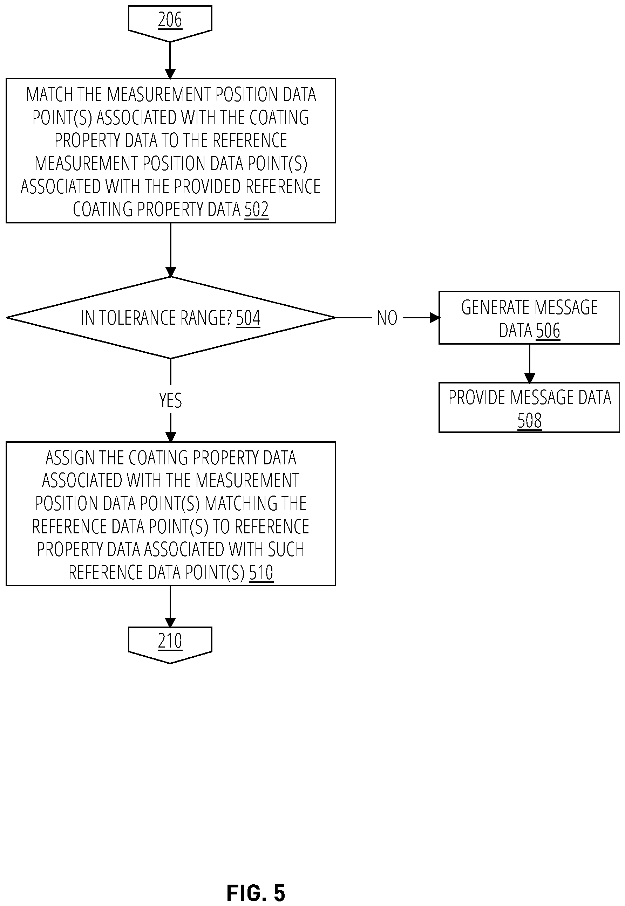

The provided coating property data may be assigned to the provided reference coating property data based on the measurement position data point(s) associated with the coating property data and the reference measurement position data point(s) associated with the reference coating property data. The coating property data may be assigned to the reference coating property data as described in the context of FIG. 5. The assigned data may be linked to the vehicle identifier. This way assigned data may be gathered based on the vehicle identifier, for example upon determination of the property of the coating. Assigning the coating property data to the reference coating property data may include matching the coating measurement position data point(s) to the reference measurement position data point(s). Matching may include matching at least a set of coating measurement position data points to at least a set of the reference measurement position data points. Matching may include determination of an absolute distance between the coating measurement position data points and the reference measurement position data points. The determined absolute distance may be compared with a given distance tolerance range or threshold to determine whether the coating measurement position data points match to the respective reference measurement position data points with a sufficient degree of accuracy, e.g. with a deviation being within the tolerance range and/or being below the given threshold. Matching of the measurement position data points with the reference measurement position data points allows to reliably assign the coating property data associated with the measurement position data points to reference coating property data in a reliable manner irrespective of the order of the acquisition of the measurement data at predefined positions on the coating.

The assigned data optionally linked to the vehicle identifier may be provided via a communication interface. The assigned data may be displayed to the user, for example within a tabular data structure. The assigned data may be stored on a data storage. The assigned data may include coating property data assigned to reference coating property data. The assigned data may include measurement position data points assigned to reference measurement position data points and coating property data assigned to reference coating property data.

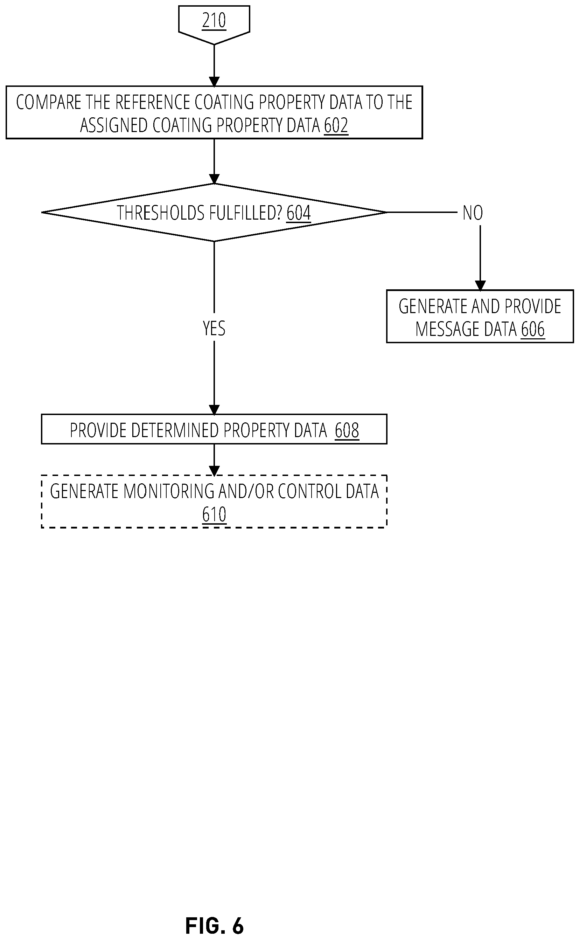

Property data associated with at least one property of the coating may be determined based on the assigned data. This step may be generally optional. Determination of the property data may include comparing the reference coating property data to the mapped coating property data. The coating property data may be compared to the reference coating property data on data point level. The property data may be determined as described in the context of FIG. 6. Determining the property data may include comparing reference color data, reference texture data, reference thickness data, reference gloss data, reference roughness data and/or reference hardness data to respectively assigned coating color data, coating texture data, coating thickness data, coating gloss data, coating roughness data and/or coating hardness data Hence, reference property data associated with a given reference measurement position data point may be compared to coating property data associated with a coating measurement position data point mapped to such reference measurement position data point. Determining the property data may include determining a color distance (also denoted as color tolerance) between the reference color data and the respectively mapped coating color data, determining a texture distance between the reference texture data and the respectively mapped coating texture data, determining a gloss distance between the reference gloss data and the respectively mapped coating gloss data, determining a roughness distance between the reference roughness data and the respectively mapped coating roughness data, determining a thickness distance between the reference thickness data and the respectively mapped coating thickness data and/or determining a hardness distance between the reference hardness data and the respectively mapped coating hardness data, The color distance may be determined using a color tolerance equation. The color distance may be determined per measurement angle associated with the measured color data and reference color data. Color tolerance equations may be selected from the Delta E (CIE 1994) color tolerance equation, the Delta E (CIE 2000) color tolerance equation, the Delta E (DIN 99) color tolerance equation, the Delta E (CIE 1976) color tolerance equation, the Delta E (CMC) color tolerance equation, the Delta E (Audi95) color tolerance equation or the Delta E (Audi2000). The determined distance may be compared to predefined distance(s). The predefined distance(s) may be included in the reference property data. The predefined distance(s) may include threshold(s) . Distance(s) above the threshold(s) may indicate an unacceptable or insufficient quality of the coating. Determination of the property data may include classifying the property/ies into acceptable and not acceptable based on the result of the comparison using a binary classifier. Classification may be performed per property. The property/ies of the coating may be rated "acceptable" if at least a part of the determined distance(s) fulfils respective predefined distance(s). The property/ies of the coating may be rated "not acceptable" if at least a part of the determined distance(s) does not fulfil respective predefined distance(s). The determination of the property data may be performed automatically after assigning the coating property data to the reference coating property data, i.e., without any interaction being necessary by the user.

The determined property data may be provided. The property data may be provided via a communication interface. The property data may be provided via the communication interface for display. Providing the determined property may include generating monitoring and/or control data for monitoring and/or controlling the property of the coating, for example as described in the context of FIG. 6. The determined property data may include determined distance(s). The property data may include a determined classifier. The property data may include data indicating deviations of the coating property data from the reference coating property data. The property data may be displayed within a 2D or 3D representation of the vehicle 114. The property data may be displayed as icon(s). For instance, the icon(s) may indicate that the quality of the coating is within predefined distance(s), outside predefined distance(s) or on the upper limits of predefined distance(s). The property data may be displayed as tabular data structure including reference coating property data, respectively mapped coating property data and respective distance(s). The property data may include coating property data and/or reference coating property data. Such reference coating property data and/or coating property data may be displayed to the user, for example within the 2D or 3D representation of the vehicle 114 or within the tabular data structure.

FIG. 3 illustrates a first example for providing reference coating data set(s) including reference coating property data associated with reference measurement position data point(s). The reference coating data set(s) may be provided for determining the property/ies of a coating, for example as described in the context of FIG. 2. The reference coating data set(s) may be provided based on measurement position data points included in the coating data associated with the coating (see FIG. 2).

The measurement position data points may be associated with coating property data. The coating property data may include a plurality of coating property data points. At least one coating property data point may be associated with a given measurement position data point. The measurement position data point may indicate the position on the coating the coating property data point(s) or measurement data used to determine the coating property data point(s) was acquired (see also FIG. 1).The measurement position data points may include Cartesian coordinates, geographic coordinates, or indoor local Cartesian coordinates. Sample distance vectors between at least a part of the measurement position data points may be determined. Sample distance vectors between all measurement position data points may be determined. The sample distance vectors may be determined based on the provided coating data.

Candidate reference coating data sets may be provided. The candidate reference coating data sets may include reference coating property data and associated reference measurement position data points. The candidate reference coating data sets may further include reference distance vectors associated with distance(s) between the reference measurement position data points. The candidate reference coating data sets may be provided via a database storing such candidate reference coating data sets. The order of block 304 and block 302 may be reversed. In other words, block 304 may be performed prior to block 302.

Absolute deviations between at least a part of the determined sample distance vectors and reference distance vector(s) may be calculated. Calculating the absolute deviations may include determining reference distance vectors based on the reference measurement position data points included in the candidate reference coating data sets. The absolute deviations may include standard deviations. The number of sample and reference distance vector(s) used for the calculation of the absolute deviation(s) may be selected such that the absolute deviation between such distance vector(s) is minimized. The absolute deviation may be minimized for given reference - sample distance vector pair and/or for a plurality of distance vectors pairs. At least a part of the determined sample distance vector(s) may not be used to calculate the absolute deviations. Such sample distance vector(s) may be associated with measurement position data point(s) not being located within a given volume, such as a given vehicle volume. This may avoid consideration of sample distance vector(s) associated with measurement position data point(s) being outside of a possible vehicle volume and hence allows to reduce the error associated with the selection of appropriate reference coating data set(s).

The minimum of the calculated deviations between the sample distance vector(s) and the reference distance vector(s) may be determined. The minimum of the calculated absolute deviations may be determined by summing up the determined absolute deviations between at least a part of the distance vectors and selecting the minimum value. The minimum of the calculated absolute deviations may be determined by comparing each deviation and selecting the combination of reference distance vectors having a minimum absolute deviation from the sample distance vectors. The minimum of the absolute deviations may be calculated by determining the root mean error of the respective absolute deviations. The minimum may be determined per candidate reference coating data set.

It may be determined if the determined minimum is within a predefined tolerance range or below a given threshold value. The tolerance range or threshold value may be included in the candidate reference coating data sets. The tolerance range or threshold value may indicate the maximum deviation of the measurement position data points from the reference measurement position data points. The tolerance range or threshold may signify the degree of error associated with the selection of the best matching reference coating data set(s) based on the measurement position data points. Since a high error may result in the use of unsuitable reference coating property data for determining the property/ies of the coating, such predefined tolerance range or threshold value may ensure that suitable (e.g. best matching) reference coating data sets are provided to allow reliable determination of the property/ies of the coating.

If the determined minimum is outside the predefined tolerance range or threshold value, message data may be generated. The message data may indicate measurement position data points associated with distance vectors being outside of the predefined tolerance range. The message data may indicate that the measurement position data point(s) is/are at least partially incorrect. The message data may include instructions to provide different coating data including coating property data and measurement position data points. The generated message data may be provided. The message data may be provided for display. The message data may be displayed to a user in the form of a message. The message may prompt the user to provide new coating property data and associated measurement position data points, for example as described in the context of FIG. 1. The method shown in FIG. 3 may be repeated using the new measurement position data points.

If the determined minimum is within the predefined tolerance range or below the threshold value, reference coating data set(s) including reference measurement position data points associated with the determined minimum may be provided. The reference coating data sets may be gathered from a database based on the reference measurement position data points associated with the determined minimum. The provided reference coating data set(s) may be used to determine the property of the coating as described in the context of FIG. 2.

FIG. 4 illustrates a second example for providing reference coating data set(s) including reference coating property data associated with reference measurement position data points. The reference coating data set(s) may be provided for determining the property/ies of a coating, for example as described in the context of FIG. 2. The reference coating data set(s) may be provided based on measurement position data points included in the coating data associated with the coating (see FIG. 2).

The reference measurement position data points may be associated with data indicating an order of the reference measurement position points. The order may signify the order of the reference measurement position data points relative to each other. The order may signify a reference measurement position data point as start point and a further reference measurement position data point as end point.

Coating data including measurement position data points may be provided, for example as described in the context of FIG. 2. Measurement position data points may include Cartesian coordinates, geographic coordinates, or indoor local Cartesian coordinates. The measurement position data points may be associated with data indicating an order of such measurement position data points relative to each other. One measurement position data point may be associated with data indicating that this data point is the start point. Another measurement position data point may be associated with data indicating that this point is the end point. Start point may refer to the first measurement position data point acquired during acquisition of the measurement data as described in the context of FIG. 1 and FIG. 2. End point may refer to the last measurement position data point acquired.

Sample distance vectors between at least a part of the measurement position data points and the measurement position data point being indicated as start point may be determined.

Candidate reference coating data sets may be provided. The candidate reference coating data sets may include reference coating property data and associated reference measurement position data points. The reference measurement position data points may be associated with order data indicating an order of the reference measurement position data points per data set relative to each other. The candidate reference coating data sets may further include reference distance vectors associated with distance(s) between a reference measurement position data point defined as start point and further reference measurement position data points. The candidate reference coating data sets may be provided via a database storing such candidate reference coating data sets. Providing the candidate reference coating data set(s) may include gathered such data set(s) based on order data associated with the measurement position points. For instance, candidate reference coating data set(s) may be gathered by matching the measurement position data point being designated by the associated order data as start point to reference measurement position data points being designated by the associated order data as start point. Use of order data to provide candidate reference coating data set(s) may improve the efficiency of the method due to the reduced number of deviations that need to be calculated.

Absolute deviations between at least part of the sample distance vector(s) and at least part of the reference distance vectors may be calculated, for example as described in the context of FIG. 3. Calculating the absolute deviations may include determining reference distance vectors based on the reference measurement position data points and associated order data. The distance vectors may be determined relative to the reference measurement position data point indicated as start point by the associated order data. The absolute deviations may include standard deviations. The number of distance vector(s) for the calculation of the deviation(s) may be selected such that the error associated with the selection of the best matching reference measurement position data points may be minimized. At least a part of the distance vectors may not be used for calculation as described in the context of FIG. 3.

The minimum of the calculated absolute deviations between the sample distance vectors and the reference distance vectors may be determined. The minimum may be determined per gathered set of candidate reference positioning data points, e.g. per reference coating data set. The minimum may be determined as described in the context of FIG. 3.

It may be determined whether the determined minimum is within a predefined tolerance range, for example as described in the context of FIG. 3. If the determined minimum is outside of such tolerance range, message data may be generated and provided, for example as described in the context of FIG. 3.

If the determined minimum is inside such tolerance range, reference coating data set(s) including reference positioning data points data associated with the determined minimum may be provided, for example as described in the context of FIG. 3. The provided reference coating data set(s) may be used to determine the property of the coating as described in the context of FIG. 2.

FIG. 5 illustrates an example of assigning coating property data to the reference coating property data based on measurement position data points associated with the coating property data and reference measurement position data points associated with the reference coating property data. Reference coating data set(s) including the reference coating property data and associated reference measurement position data may be provided based on the vehicle identifier as described in the context of FIG. 2. The reference coating data set(s) may be provided based on the measurement position data points as described in FIG. 3 or FIG. 4.

The measurement position data point(s) associated with the coating property data may be matched to the reference measurement position data point(s) associated with the provided reference coating property data. Matching may include determining absolute deviation(s) between at least a part of the measurement position data points and the reference measurement data points and minimizing the determined deviations. Calculation of the absolute deviation(s) may include calculating sample distance vectors between at least a part of the measurement position data points and calculating absolute deviations between the determined sample distance vectors and reference distance vectors included in the provided reference coating data set(s). Calculation of the absolute deviation(s) may include calculating reference distance vectors between at least a part of the reference measurement position data points and calculating absolute deviations between the determined reference distance vectors and sample distance vectors included in the provided coating data. Calculation of the absolute deviation(s) may include calculating reference distance vectors between at least a part of the reference measurement position data points, calculating sample distance vectors between at least a part of the measurement position data points and calculating absolute deviations between the determined reference distance vectors and the determined sample distance vectors. Calculation of the absolute deviation(s) may include calculating the deviations between sample distance vectors included in the provided coating data and reference distance vectors included in the provided reference coating data set(s). Absolute deviations between vectors may be determined as described in the context of FIG. 3 and FIG. 4. The absolute deviation(s) may be calculated in order to determine the relative position between at least two positioning data points.

Matching may include minimizing determined absolute deviations between at least a part of the measurement position data points and the reference measurement data points. The absolute deviation(s) may have been already calculated, for example as described in the context of FIG. 3 and FIG. 4. The absolute deviation(s) may be included in the provided coating data and reference coating data set.

The absolute deviation(s) may be minimized as described in the context of FIG. 3 and FIG. 4. Minimizing the absolute deviations may include determining the similarity between a coating measurement position data point and one or more reference measurement position data points or vice versa. Matching may include permuting the order of the coating measurement position data points and determining the absolute distance to each reference measurement position data point per reference coating data set. Matching may include determining the minimum absolute deviation between sample distance vector(s) and reference distance vector(s). An example of matching coating measurement position data points to reference measurement position data points is illustrated in the simplified example shown in Table 1. Table 1 measurement position data points, reference measurement position data points and order data associated with the respective position data points. For a given order of measurement position data points, the absolute distance between the measurement position data points and the reference measurement position data points may be determined per data point. Afterwards, the order of the measurement position data points may be permuted while keeping the order of the reference measurement position data points constant or *vice versa* and the absolute distance after permutation may be determined for one or more data point(s). Comparison of such absolute distances allows to determine the minimum absolute deviation between the measurement position data points and the reference measurement position data points. The minimum absolute deviation may be used to match the measurement position data points to the respective reference measurement position data points.

**Table 1: example of order data associated with the coating measurement position data and reference position data respectively, coating measurement position data and reference positioning data**

| | | | | |
|---|---|---|---|---|
| measurement order data | 1 | 2 | 3 | 4 |
| coating measurement position data | (1,4) | (2,6) | (5,7) | (8,10) |
| reference measurement position data | (2,6) | (8,10) | (5.1,7) | (1,4) |

Table 2 shows a simplified example of the matching result.

**Table 2: result of matching of coating measurement position data points to reference measurement position data points**

| | | | | |
|---|---|---|---|---|
| reference measurement position data point | (2,6) | (8,10) | (5,7) | (1,4) |
| coating measurement position data point | (2,6) | (8,10) | (5.1,7) | (1,4) |
| Minimized deviation | (0,0) | (0,0) | (0.1, 0) | 0,0) |

It may be determined if the minimized absolute deviation between the measurement position data points and the reference measurement position data points is within a given tolerance range. The determination may be performed per measurement position data point - reference measurement position data point pair. The tolerance range may be included in the provided reference coating data set(s). In the example illustrated above, e.g. in Table 2, it may be determined whether the absolute deviations between the coating measurement position data points and the reference measurement position data points are within the given tolerance range. If the minimized absolute deviation is not within the given tolerance range, message data may be generated and provided for display (see blocks 512, 514). The message data may indicate that the measurement position data points are outside the given tolerance range and are not sufficiently similar to the reference measurement position data points to allow reliable determination of the property/ies of the coating (see block 514). The message may prompt the user to repeat acquisition of measurement position data points and associated measurement data. Since the coating property data may be highly dependent on the measurement location used to acquire such data or used to acquire measurement data used to determine such data, using given tolerance ranges may allow to obtain reliable results when determining the property of the coating, since such tolerance ranges ensures that the deviation of the measurement position data points to the reference measurement position data points does not significantly influence the property of the coating.

If the minimized absolute deviation is within the given tolerance range, the coating property data may be assigned to the reference coating property data or vice versa based on the matching of the coating measurement position data points to the reference measurement position data points. Assigning may include grouping the coating property data point associated with a given measurement position data point with a reference coating property data point associated with a reference measurement position data point matching the measurement position data point. The coating property data may be assigned to the reference coating property data on data point level. The coating property data may be assigned to the reference coating property data on data point level based on the property data type. Each data point included in the property data (e.g. the coating property data and the reference coating property data) may include a key-value pair. The key may define the property data type and the value may define the property data. Based on the keys and the matched position data, the coating property data points may be assigned to the reference coating property data points. For instance, coating color data included in the coating property data may be assigned to reference coating color data included in the reference coating property data while coating texture data included in the coating property data may be assigned to reference texture data included in the reference coating property. This ensures that the coating property data can be reliable compared to respective reference coating property data irrespective of the order used to acquire the coating property data or used to acquire measurement data used to determine the coating property data and irrespective of the property data types included in the coating property data and the reference coating property data . The assigned data may be provided for determining property data associated with property/ies of the coating, for example as described in the context of FIG. 2.

FIG. 6 illustrates an example of determining property data associated with at least one property of a coating present on at least a part of a surface of a vehicle.

Determining the property data may include determining deviation(s) between the coating property data and the respectively assigned reference coating property data. Determining the deviation(s) may include comparing coating property data types with the respectively assigned reference coating property data types. For instance, coating color data may be compared to assigned reference coating color data, color texture data may be compared to assigned reference texture data, coating gloss data may be compared to assigned reference gloss data, coating thickness data may be compared to assigned reference thickness data, coating roughness data may be compared to assigned reference roughness data and/or coating hardness data may be compared to assigned reference roughness data. The deviation(s) may be determined on data point level. Comparing the coating color data with the respectively assigned reference color data may include calculating color difference(s) or color tolerances (e.g. known as dE) between the coating color data and the reference color data. Color tolerances may be determined using a color tolerance equation, such as the Delta E (CIE 1994) color tolerance equation, the Delta E (CIE 2000) color tolerance equation, the Delta E (DIN 99) color tolerance equation, the Delta E (CIE 1976) color tolerance equation, the Delta E (CMC) color tolerance equation, the Delta E (Audi95) color tolerance equation or the Delta E (Audi2000) color tolerance equation.

Determining the property data may further include comparing the deviation(s) to predefined threshold value(s). The predefined threshold value(s) may be included in the reference coating data set(s). The predefined threshold value(s) may be included in the reference coating property data. The deviation(s) may be compared on property type level. Based on the comparison, the respective property type may be classified as "acceptable" or "non-acceptable". For example, the predefined color threshold(s) may be applied to the determined color difference(s) to determine whether the color difference(s) between the coating color data and the reference color data are below the predefined threshold value(s) for dE. If the determined color difference(s) are below the threshold value(s), the color property may be classified as "acceptable". Otherwise, the color property may be classified as "not acceptable". The classifier may be a binary classifier discriminating between acceptable property or property type and non-acceptable property or property type. The classifier may relate to the deviation(s) being above or below given threshold value(s). This may allow to determine whether the property/ies of the coating fulfils given or predefined property/ies.

The property/ies or property type(s) is/are rated as "acceptable", the property/ies or property type(s) may be provided as property data. The property data may include the property/ies or property type(s) associated with deviation(s) with respect to the assigned reference property/es or property type(s) that are below the threshold value(s) and the determined deviation between the coating property data and the assigned reference coating property data. The property data may further include position data point(s) and/or deviation(s) between the position data point(s). Providing the property data may include providing the determined property data via a communication interface. Providing the determined property data may include providing the determined property data via a communication interface for display.

Monitoring data for monitoring the property of the coating may be generated based on the determined property data. The monitoring data may be generated based on the deviation of the coating property data from respectively assigned reference coating property data. This deviation may indicate or signify the condition or state of the coating. The monitoring data may include data indicating new provisioning of coating property data to reevaluate the property of the coating. The data indicating new provisioning may include the vehicle identifier and a time point for providing new coating property data. The data may further include the determined property. By generating monitoring data based on the determined property of the coating, the property of the coating may be more reliably monitored. This way, coating maintenance may be performed in a more efficient manner, avoiding repair of large areas of the coating and allowing to reduce the environmental impact associated with the maintenance by reducing the amount of coating materials and energy consumption as well as the waste generation associated with the maintenance.

Control data for controlling the property of the coating may be generated based on the determined property data. The control data may be generated based on the deviation of the coating property data from respectively assigned reference coating property data. The control data may include process data associated with a process for modifying the coating. Modifying the coating may include removing the coating and recoating the surface to prepare a new coating, Modifying the coating may include overcoating the coating to prepare a new coating on the existing coating. The process data may include a process identifier associated with the process. By generating control data based on the determined property of the coating, the property of the coating may be adjusted to match a given target property, e.g. the property of a reference coating associated with the reference coating property data. This way, it may be ensured that the coating fulfils required property/ies.

The present disclosure has been described in conjunction with preferred embodiments and examples as well. However, other variations can be understood and effected by those persons skilled in the art and practicing the claimed invention, from the studies of the drawings, this disclosure and the claims.

Any steps presented herein can be performed in any order. The methods disclosed herein are not limited to a specific order of these steps. It is also not required that the different steps are performed at a certain place or in a certain computing node of a distributed system, i.e. each of the steps may be performed at different computing nodes using different equipment/data processing.

As used herein "determining" also includes "initiating or causing to determine", "generating" also includes "initiating and/or causing to generate" and "providing" also includes "initiating or causing to determine, generate, select, send and/or receive". "initiating or causing to perform an action" includes any processing signal that triggers a computing node or device to perform the respective action.

In the claims as well as in the description the word "comprising" or "including" or similar wording does not exclude other elements or steps and shall not be construed limiting to the elements or steps lined out. The indefinite article "a" or "an" does not exclude a plurality. A single element or other unit may fulfill the functions of several entities or items recited in the claims. The mere fact that certain measures are recited in the mutual different dependent claims does not indicate that a combination of these measures cannot be used in an advantageous implementation or further elements may be included.

Providing in the scope of this disclosure may include any interface configured to provide data. This may include an application programming interface, a human-machine interface such as a display and/or a software module interface. Providing may include communication of data or submission of data to the interface, in particular display to a user or use of the data by the receiving node, entity or interface.

All terms and definitions used herein are understood broadly and have their general meaning.

## Claims

1. A method for monitoring and/or controlling the property of a coating present on at least a part of the surface of a vehicle during manufacturing of the vehicle and/or during use of the vehicle, the method comprising the steps of:
• providing a vehicle identifier associated with the vehicle,
• providing coating data associated with the coating, wherein the coating data includes coating property data associated with the property of the coating and measurement; position data point(s) associated with the coating property data;
• providing reference coating data set(s) associated with reference coating(s), wherein each reference coating data set includes reference property data associated with a property of a reference coating and reference measurement position data point(s) associated with the reference coating property data;
• assigning the coating property data to reference coating property data based on the measurement position data point(s) associated with the coating property data and the reference measurement position data point(s) associated with the reference coating property data;
• providing the assigned data optionally linked to the vehicle identifier for determining property data associated with at least one property of the coating.

2. The method of claim 1, wherein the measurement position data point(s) indicate(s) one or more position(s) on the coating used to acquire the coating property data or used to acquire measurement data for determining the coating property data.

3. The method of claim 1 or 2, wherein the reference measurement position data point(s) indicate(s) one or more position(s) on the coating used to acquire the reference coating property data or used to acquire measurement data for determining the reference coating property data.

4. The method of any one of claims 1 to 3, wherein the reference coating data set(s) is/are provided based on the vehicle identifier and/or based on the coating measurement position data.

5. The method of any one of claims 1 to 4, wherein the provided reference coating data set(s) is/are associated with vehicle type(s) matching the vehicle type the vehicle is associated with and/or is/are associated with a coating matching the coating present on the surface of the vehicle.

6. The method of any one of claims 1 to 5, wherein assigning the coating property data to the reference coating property data includes
• matching the measurement position data point(s) to reference measurement position data point(s), in particular per reference coating data set, by minimizing the deviation(s) between the measurement position data point(s) included in the coating data and the reference measurement data point(s) included in the reference coating data set(s), and
• assigning the coating property data associated with the measurement position data point(s) matching the reference data point(s) to reference property data associated with such reference data point(s).

7. The method of any one of claims 1 to 6, wherein the coating property data is assigned if at least part of the position data point(s) match(es) to at least a part of the reference position data point(s), in particular per reference coating data set.

8. The method of any one of claims 1 to 7, wherein the coating property data is assigned if deviation(s) between at least a part of the measurement position data point(s) and at least a part of the reference measurement position data point(s) is/are below a given threshold value(s).

9. The method of any one of claims 1 to 8, wherein at least a part of the coating property data is not assigned to the reference coating property data if the deviation(s) between at least a part of the measurement position data point(s) and the reference measurement position data point(s) is/are above given threshold value(s).

10. The method of claim 9, wherein data indicating non-assignment of the coating property data to reference property data is generated and provided.

11. The method of any one of claims 1 to 10, further including a step of determining - based on the assigned data - the property data, linking the property data to the vehicle identifier and providing the determined property data linked to the vehicle identifier.

12. The method of claim 11, wherein the property data is determined by determining deviation(s) of the reference coating property data to the assigned coating property data, in particular wherein the deviations are determined on data point level.

13. An apparatus for monitoring and/or controlling the property of a coating present on at least a part of the surface of a vehicle during a manufacturing process and/or during use of the vehicle, the apparatus comprising:
• an identifier providing interface configured to provide a vehicle identifier associated with the vehicle,
• a data providing interface configured to provide
- based on the vehicle identifier - coating data associated with the coating, wherein the coating data includes coating property data associated with the property of the coating and coating measurement position data point(s) associated with the coating property data,
- reference coating data set(s) associated with reference coating(s), wherein each reference coating data set includes reference coating property data associated with a property of a reference coating and reference measurement position data point(s) associated with the reference coating property data;
• an assignor configured to assign the coating property data to the reference coating property data based on the measurement position data point(s) associated with the coating property data and the reference measurement position data point(s) associated with the reference coating property data;
• a data providing interface configured to provide the assigned data for determining property data associated with the property of the coating.

14. A system for monitoring and/or controlling the property of a coating present on at least a part of the surface of a vehicle during a manufacturing process and/or during use of the vehicle, the system comprising:
• at least one measurement device configured to provide coating data associated with the coating, wherein the coating data includes coating property data associated with the property of the coating and measurement position data point(s) associated with the coating property data;
• at least one processor configured to
- provide reference coating data set(s) associated with reference coating(s), wherein each reference coating data set includes reference property data associated with a property of a reference coating and reference measurement position data point(s) associated with the reference coating property data;
- assign the coating property data to reference coating property data based on the measurement position data(s) associated with the coating property data and the reference measurement position data point(s) associated with the reference coating property data;
- provide the assigned data for determining the property of the coating.

15. Use of a property of a coating present on at least one surface of a vehicle as determined according to the method of any claim 11 or claim 12 for monitoring and/or controlling the property of a coating being present on at least a part of the surface of a vehicle, in particular during manufacturing of the vehicle and/or during use of the vehicle.
